# EUROPEAN PATENT SPECIFICATION

(11) **EP 2 665 431 B1**
(45) Date of publication and mention of the grant of the patent: **07.06.2023**
(21) Application number: 12702308.3
(22) Date of filing: 20.01.2012
(51) Int. Cl.: A61B 17/34, A61M 37/00

(54) **DRUG DELIVERY TECHNOLOGY**
WIRKSTOFFFREISETZUNGSVERFAHREN
TECHNOLOGIE D'ADMINISTRATION DE MÉDICAMENTS

(30) Priority: 21.01.2011 GB 201101087
(43) Date of publication of application: 27.11.2013
(73) Proprietor: Avaxzipen Limited, Abingdon, Oxfordshire OX14 4SA (GB)
(72) Inventor: POTTER, Charles, Oxon OX29 7SG (GB); WATSON, John, Oxon OX29 7SA (GB)
(74) Representative: Stratagem IPM Limited
(86) International application number: PCT/GB2012/000049
(87) International publication number: WO 2012/098356

(56) References cited:
- NL-A- 8 902 283
- US-A- 4 846 793
- US-A- 6 102 896

## Description

### FIELD OF THE INVENTION

The present invention relates to a novel drug delivery technology. It is also disclosed, although it is not part of the invention, a method of delivering a solid drug formulation, for example, in the form of a solid drug needle or implant, through the skin by pre-cutting the skin to ease the path of the solid drug through the skin. The invention also relates to a solid dose injector device, for delivering the solid drug formulation, the device including an actuation means and a blade for breaking the outer layer of the skin to allow the solid drug formulation to more easily penetrate the skin to the desired depth. It also relates to an injection device that comprises a reusable actuation device and a disposable cassette assembly containing the solid drug formulation and a blade; and cassettes for use with the actuation device.

### BACKGROUND TO THE INVENTION

One route of administration for therapeutic compounds is through the skin. The skin is also one of the more efficient routes for delivery of a therapeutic compound when compared to other standard delivery routes such as oral or pulmonary delivery. Therapeutic compounds in the form of solid drug formulations/implants have been administered through the skin via a hollow needle and trocar apparatus such as that disclosed in EP0139286 (Sumitomo Chemical Co Limited). However, this apparatus was designed to be used by a trained healthcare professional and it is clearly unsuitable for regular use or for self-administration. In addition, as can be seen from the figures therein, the needle is inserted deep into the tissue before the drug is pushed out beyond the end of the needle using the trocar.

Ipsen (US 7,798,988) also discloses a needle and trocar delivery system that is inserted deep into the tissue. In this embodiment the trocar is held steady while the needle is withdrawn leaving the solid drug formulation in place in the tissue rather than the trocar pushing the beyond the end of the needle.

More recently a significant amount of research has focussed on the development of sophisticated needle-free injection systems for both liquid and solid drugs. For example, solid form drugs have been successfully administered with the PowderJect system, which uses a compressed gas source to accelerate powdered drugs to a velocity at which they can penetrate the outer layers of the skin. This system typically employs powdered drug particles of less than 100 microns in diameter, which require a velocity of several hundred metres per second in order to penetrate human tissue.

It has also been shown in the past that solid rods or splinters of a therapeutic compound can be pushed, at a relatively low velocity, into the skin without the requirement for a needle. US 5,542,920, US 6,117,443 and US 6,120,786 (Cherif Cheikh) all disclose needle-needle-free parenteral introduction devices for administering solid drug formulations. The medicament is made in the form of a solid needle having a pointed end that has sufficient structural integrity to penetrate the skin. Similarly, US 6,102, 896 (Roser) is primarily directed to a disposable injector device for injecting solid drug needles. NL8902283 (Nedap) is directed to implant introduction devices in which a rounded implant is ejected from a needle via an internal rod.

The present applicant has also developed a needle-free solid drug delivery device which is described in full in international patent applications PCT/EP02/10394 and WO 2004/014468. The content of these applications is included by reference. A more recent application, WO2006/082439, in the name of the present applicants, discloses a device having a skin piercing element which may be a blade, such as a needle or a lancet. However, the blade is used in a conventional manner, i.e. to pierce the skin to perform a liquid injection or to collect a blood sample. The application also discloses an alternative embodiment, in the form of a needle-free solid drug injection device which has a skin piercing element in the form of a projectile that is pushed out of the device and into the skin to cut a path for a solid drug formulation that follows immediately behind.

The needle-free solid drug injection systems of the prior art, including those that utilise solid drug rods or needles, require the solid drug formulation to be propelled at a velocity sufficient to break the tough outer layer of the skin and thus the solid drug formulation must be sufficiently robust to withstand the forces required to achieve initial skin penetration. Ensuring a sufficiently robust formulation that can penetrate different skin locations (arm, stomach, thigh etc) and skin types (male / female, child / adult / geriatric, black African / white Caucasian / Asian etc) can be a challenge both to develop and also to manufacture at a commercial scale.

One of the primary motives for the development of needle-free solid drug injector devices has been the desire to reduce the pain and/or fear associated with the use of needles to inject medicaments. Thus research in this field has focussed on the development of devices that permit the delivery of solid drug formulations without the use of needles.

### SUMMARY OF THE INVENTION

The invention provides a solid dose injection device in accordance with the claims herein, comprising at least:
i) a casing (22),
ii) an actuation mechanism capable of generating a force,
iii) a blade (14),
iv) a solid drug formulation (13); and v) and a means (10) for pushing said blade (14) and said solid drug formulation (13);
wherein the solid dose injection device is suitable to be actuated so that upon actuation of the device the actuation mechanism capable of generating a force acts on the means for pushing to push the blade (14) from the casing (22) to penetrate the skin of the human or animal body to a predetermined depth wherein, following skin penetration by the blade (14), the means (10) for pushing continues to push the solid drug formulation (13) out of the casing (22) and into the human or animal body; wherein the solid drug formulation (13) is pushed into the body to a depth that extends beyond the depth of the blade (14); and wherein said solid drug formulation (13) comprises a cutting element to facilitate its passage through the skin.

It is disclosed herein that in order to enable a solid drug formulation to more easily penetrate the skin it is beneficial to make a shallow cut in the tough outer layer of the skin prior to pushing the solid drug formulation into the body through the cut. The cut may be very shallow in the skin and the solid drug formulation still needs to create a path through the underlying tissue but its passage through the main barrier, the outer layer of the skin, has been eased by breaking the skin prior to pushing in the solid formulation. It is believed that the use of such a shallow cut will be acceptable to the user as the sensation should be minimal.

The inventors have found that a cut depth of up to 4mm is sufficient to create an opening to allow the solid drug formulation to more easily penetrate the skin. However, it is believed that a cut of about 3 mm is sufficient and that less than 2 mm may be adequate. Thus, the cut may 4mm or less in depth, but may be less than 3mm or less than 2mm or between about 2 and 3mm. The exact depth of the required cut will depend on the injection site chosen, as the thickness of the skin's tough outer layer varies at different locations on the body. Preferably a cut of between 2mm and 4mm in depth is used.

A cut of up to 10 mm in depth may be used to help a solid formulation penetrate to a greater depth in the tissue, particularly if the formulation is not sufficiently strong to enable it to penetrate the tissue itself.

As described above, the present disclosure includes devices configured for either manual or automatic insertion of the blade.

Thus, the solid drug injection device may include a blade that is pushed into the skin by the user to a predetermined depth before the device is actuated to automatically push the solid drug formulation out of the device and into the cut made by the blade.

One of the main motives of recent research into solid drug delivery methods and devices has been to avoid the use of needles. Thus, in the context of the present disclosure it is preferred that any devices or methods use a blade that is not visible to the patient prior to use. Thus, the blade is held within a casing and pushed from the casing upon injection, thus preferably the blade is slidably mounted in the casing.

A device configured for manual insertion of the blade, may include a blade that is hidden by a shroud or cover and as an injection device is pushed against the skin the shroud is pushed back and the blade enters the skin. Alternatively, the device is configured such that blade is hidden by a shroud or cover and as the device is pushed against the skin a snap means or trigger releases the shroud and the resultant pressure against the skin pushes the blade into the skin and subsequently pushes the solid formulation into the skin. This mechanism, which is not part of the presently claimed invention, may be difficult to design to reliably ensure that the blade is inserted prior to the formulation and that the user cannot perform a `poor injection' by pressing hard enough to enable the needle to be inserted but not continue with sufficient force to inject the solid formulation.

However, the injection device, in accordance with the invention as claimed and that incorporates automatic insertion of both the blade and the solid drug formulation, is advantageous, particularly if the injection is to be self-administered.

Following injection the blade is withdrawn from the skin, thus the device may include a mechanism for retracting the blade, advantageously this is an automatic mechanism. The automatic mechanism may be a helical spring which draws the blade and the ejector pin (means for pushing) into the cassette (casing) at the end of the injection process. If the blade is manually withdrawn from the skin then preferably it will be automatically covered or shrouded so that the blade does not pose a sharp hazard.

Advantageously, the device can be actuated by a single push from the user, meaning that the solid drug formulation can be readily self-administered. The device may comprise a single means for pushing first the blade and then solid drug formulation into the body. Solid dose injection devices according to any aspects of the present invention may take the form of a single unit incorporating all the elements needed to inject the solid drug formulation, i.e. an actuation mechanism, blade and means for pushing the solid drug formulation into the body of a patient. Such a device may be disposable or reusable. Alternatively, they may be multi-part devices the parts being assembled prior to performing the injection.

The injection device may comprise a reusable actuation mechanism and a disposable cassette assembly that is used with the actuation device. Examples of suitable actuation mechanisms and cassettes include those described in the inventors' earlier applications, including WO 2004/014468 and WO 2006/082439. Thus, when assembled, the device comprises two main parts, the first being the actuation device that comprises a casing that houses the reusable actuation mechanism and a second part comprising a disposable cassette which houses the blade, solid drug formulation and a means for pushing the blade and solid drug formulation.

The actuation mechanism drives the means for pushing the solid drug into the body. However, in the context of the present invention, it will be apparent to the skilled man, that the exact configuration of actuation mechanism is not important as long as it acts on the means for pushing with a force sufficient to perform the injection. The actuation mechanism capable of generating a force may be a spring-powered mechanism. Suitable mechanisms are described in WO 2004/014468 and WO 2006/082439.

As set out above, in order to provide a user friendly device, the means for pushing the blade and solid drug into the body may be in the form of a single means, for example, an ejector pin to which the blade is coupled via a blade mounting. Alternatively, separate means for pushing the blade and then solid drug may be utilised, although this may complicate the injector device and is likely to necessitate more than one step by the user in order to perform the injection.

The device may be configured such that the movement of the blade and the ejector pin are coupled during the first part of the injection such that both the blade and the ejector pin move forward until the blade is fully inserted into the skin. Then, during the second part of the injection, the ejector pin and blade are uncoupled such that the ejector pin continues to move forward to push the drug into the cut made by the blade. Thus the device may be configured such that the ejector pin and the blade mounting are releasably coupled.

The means for pushing the blade and solid drug formulation into the body may be configured such that the blade is mounted in a blade mounting that is releasably coupled to the ejector pin such that upon actuation of the device the ejector pin and blade mounting move forward until the blade is pushed out of the casing, the ejector pin is released from the blade mounting, and the ejector pin continues to move forwards pushing the solid drug formulation out of the casing. Thus a single means is utilised to push both the blade and then solid drug formulation into the skin. The casing may include a stop means such that the blade mounting is stopped. The end of the casing may act as the stop means.

The ejector pin and blade mounting may be releasable coupled via resilient arms. The resilient arms may be on the blade mounting. The resilient arms are held in a fixed position by the casing while the blade is pushed from the casing and, upon skin penetration by the blade, the resilient arms are permitted to flex and uncouple from the ejector pin, such that the ejector pin continues to push the solid drug formulation into the body. The casing may include a wider section to permit the arms to flex once the blade is inserted.

Alternatively, the ejector pin and blade are releasably coupled via resilient arms on the ejector pin. The ejector pin may be mounted in an ejector pin mounting that comprises resilient arms and the ejector pin and blade mounting are releasable coupled via the resilient arms on the ejector pin mounting. The casing may include a wider section to permit the arms to flex once the blade is inserted.

The term ejector pin is intended to cover a pin, piston, and rod or like member which functions to push the solid drug formulation from the casing.

As described above, by use of the device, the cut made by the blade creates an opening in the skin thus easing the path of the solid drug through the barrier created by the outer layer of the skin. The inventors have found that various configurations of blade may be used. The cut may be a straight cut of, for example, 1 mm in length, thus a blade with a relatively flat profile may be utilised. The blade may have a pointed, flat, curved or other shaped end which will vary the depth of the cut. The blade may be a curved blade.

Advantageously the blade is a hollow blade such as a hollow needle. This is advantageous as the solid drug formulation may be pushed through the lumen of the needle into the skin. If the solid drug formulation is circular in cross section then it might be easier to use a curved blade, similar (or identical) to the end of a hypodermic needle - particularly because a hypodermic needle has been designed to penetrate skin easily. Ideally the blade selected is sharp so that it penetrates the skin easily and with a minimum sensation. Modern hypodermic needles are made from steel and they are designed with five cutting edges to enable easy penetration of the skin with minimum discomfort to the patient. Plastic needles have been investigated but it is difficult to ensure they are manufactured sharp enough to penetrate the skin with a low sensation and it is unlikely that a plastic needle can have the same fine wall thickness as a steel needle.

When a traditional hypodermic needle is pushed fully into the skin it creates a full circumference cut. It is thought that this may be disadvantageous as it can create a plug of tissue which could impede the passage of the solid drug formulation into the skin. In embodiments of the present invention it is preferred that the blade is adapted to create a straight or arced cut in the skin, rather than a full circumference cut. Thus the end of a hypodermic needle may be used as the blade in the embodiments of the invention but it does not need to be pushed fully into the skin such that a full circumference cut is produced. It may be inserted such that an arced cut is produced, for example, of approximately 270 degrees. Advantageously an arced cut can create a 'flap' of skin through which the formulation can be pushed.

As described above the cut made by the blade need not be very deep, it is sufficient to make a relatively superficial cut in the skin to create the start of a pathway for the solid drug to move through. Thus it will be apparent to the skilled man that the depth of the cut can be controlled, for example, by using a needle of limited length and/or by locating a stop means at an appropriate position in the casing such that the length of the blade extending from the casing into the body of the patient is limited. Thus preferably the blade length is less than or equal to 4mm, more preferably less than 3mm or, preferably about 2mm, as it is believed that about 2mm is sufficient. As stated earlier the exact blade length required may depend on the injection site to be used, the thickness of the tough outer layer of the skin at that site, the age, gender and ethnicity of the patient.

The blade does not necessarily have to produce a cut the same size as the solid drug formulation. The solid drug formulation also further cuts the skin to allow the solid formulation to be pushed into the underlying tissue.

The device of the present invention may be configured such that the solid drug formulation is pushed out of the casing and into the body such that its passage into the body is guided by the blade. Thus the solid drug formulation may be pushed along the length of the blade. Thus, the profile of the solid drug formulation may match the profile of the blade. Thus, a curved blade would be used with a curved or cylindrical solid drug formulation.

For most drug delivery applications it is envisaged that the solid drug formulation will be pushed into the skin beyond the depth of the blade/cut. This is to ensure that drug is delivered to the appropriate depth in the body and also that the drug stays in the body and doesn't leak or fall out of the shallow cut. Thus, the devices of the invention are configured such that the solid drug formulation is pushed into the body to a depth that extends beyond the depth of the blade. As will be apparent to the skilled man, the devices of the invention can be adapted to deliver solid drug formulations to different predetermined depths by varying a number of different parameters, including the force applied by the ejector pin to the solid drug formulation, the length of the ejector pin, the shape and profile of the solid drug formulation and/or the materials selected for the solid drug formulation.

Advantageously, the device is configured such that the ejector pin can be pushed out beyond the end of the blade. Preferably the device is configured such that the ejector pin maintains contact with the solid drug formulation during the injection. Thus the solid drug form is pushed into the cut made by the blade and then pushed further into the underlying tissue by the force of the ejector pin.

The solid drug formulation of the solid dose injection device invention further comprises a cutting element to facilitate its passage through the skin beyond the cut made in the skin by the blade. This may take the form of a sharp point or an oblique edge at the skin penetrating face, it may, for example, be configured to match the end of the blade, for example, the profile of the end of a hypodermic needle.

Solid drug formulations such as those used with prior art needle-free injection devices, for example, solid drug rods, drug splinters, implants and pioneer projectiles immediately followed by the drug in the form of beads, granules, powder or in a contained state are all suitable for use in the practice of the invention, providing, of course, that they can be pushed by the means for pushing into the cut made by the blade. As set out above, the blade may be used to guide the passage of the solid drug formulation into the body and thus, the solid drug formulation is in a form that can easily be pushed along the blade. For example, if a curved or cylindrical blade is used it is advantageous to use a drug formulation that is curved at the face that contacts the blade. Similarly, if a substantially flat blade is used a solid drug formulation that is substantially flat at the face that contacts the blade is preferred.

Thus, if the blade is a hypodermic needle it is preferable to use a solid drug formulation that is cylindrical in cross-section and that has a skin penetrating face that matches the profile of the pointed end of the needle.

One of the key advantages of the methods and devices of the present invention is that because the blade opens a pathway through the tough outer layer of the skin the path of the solid drug formulation into the body is eased. This gives much greater flexibility in terms of selecting the appropriate materials for the formulation. For example, the formulation need not be as mechanically strong or as sharp as those disclosed in the prior art.

Advantageously, the solid dose formulation that is pushed into the skin through a cut does not require the same mechanical properties as one that has to penetrate the tissue without a cut. If a cut is not made in the skin then a pharmaceutical formulation of approx 0.9 mm in diameter with a pointed tip is likely to require a peak force in excess of 25 Newtons to penetrate the skin. This may be close to or above the mechanical strength of the material, meaning that some injections may not always be successful because the formulation crushes before penetration of the skin occurs. If a cut is made in the skin then the same pharmaceutical formulation can be pushed into the skin through the cut with a peak force of less than 15 Newtons. The required force still depends on the shape of the tip and the diameter of the formulation but significantly lower forces are required for penetration when a cut has been made in the skin. As lower forces are required then the mechanical properties of the formulation are less.

As disclosed in their earlier applications, the inventors have found that it is advantageous in the practice of the present invention for the ejector pin to maintain contact with the solid drug formulation during delivery. This means that the ejector pin acts directly on the solid drug formulation by means of pushing it forward rather than by striking it at velocity during device actuation. This pushing motion results in minimal stress to the solid drug formulation. It also means that the solid drug formulation does not need to have the mechanical strength to withstand an impact from the ejector pin. Preferably, there is a stop means for the ejector pin such that the solid drug formulation is pushed to the same depth in the tissue every injection.

As discussed above, because the pathway through the tough outer layer of the skin has been eased by the use of the cutting blade the solid drug formulation can be pushed with lower force and at relatively low velocity into the body. Preferably the solid drug formulation is pushed into the skin through the cut with a peak force of less than 15 Newtons. The required force still depends on the shape of the tip and the diameter of the formulation but significantly lower forces are required for penetration when a cut has been made in the skin. As lower forces are required then the mechanical requirements of the formulation are less stringent.

Advantageously the devices of the invention are configured such that the solid drug formulation is pushed into the cut at a low velocity and thus the devices of the present invention may be configured to push the solid drug formulation at low velocity. By low velocity is meant less than 100m/s. The velocity may be less than 10m/s, or than 5m/s or in the order of a few m/s.

Since the solid drug formulation is pushed at a low velocity rather than fired at a high velocity, it is possible to ensure that the dosage is always delivered to the correct (and same) depth under the skin. This means that the system can be used on different skin types and skin locations and the dosage will still be delivered to the same depth.

At the end of the injection the blade must be removed from the skin, this could be done manually but preferably the devices of the invention incorporate a blade retraction means, more preferably the blade retraction means is automatic. Preferably the blade retraction means withdraws the blade from the skin and into the casing so that there is no risk of a sharps injury. Ideally this is done automatically by a mechanism present in the device, for example, WO2006/082439 provides details of a suitable mechanism. If the blade is automatically removed from the skin then the removal must not draw the solid formulation from the skin, although this is unlikely if the solid formulation is pushed beyond the end of the blade.

In embodiments, the devices according to the present invention incorporate a mechanism for withdrawing from the skin the means for pushing the solid drug formulation at the end of the injection. Thus, the devices may include a means for withdrawing the ejector pin, this may be an automatic means that retracts the ejector pin. A single retraction means, for example, in the form of a helical spring, may be used to retract both the ejector pin and the blade.

The injection device may be configured such that it is obvious to the user when it has been used, for example, by including an indicator means in the cassette. For example the injection device or cassette may include an indicator means that shows that it has been used. Preferably, it is not possible to reuse the device, thus it may include a disabling mechanism, more preferably an automatic disabling mechanism that prevents the device from being re-used. The device may include an indicator means and a disabling mechanism; preferably these are included in the cassette. Suitable mechanisms include those disclosed in WO 2006/082439 A1,

### BRIEF DESCRIPTION OF THE DRAWINGS

Figure 1 shows a cross sectional side view of a solid dose injection device according to the present invention, in this embodiment in the form of a cassette that is used with an actuation means. Figure 1a shows the cassette prior to actuation; Figure 1b shows the blade moving forward; Figure 1c shows the solid drug formulation injection; and Figure 1d shows the cassette following injection with the blade retracted.
Figure 2 shows another embodiment of the invention in which a cassette includes resilient arms on the ejector pin that releasably couples the blade mounting to the ejector pin.
Figure 3 shows another embodiment of a cassette that includes a wire hairpin that releasably couples the blade mounting to the ejector pin.

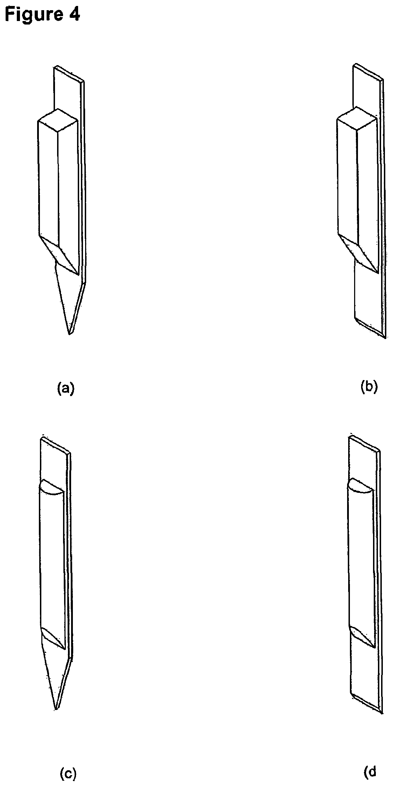

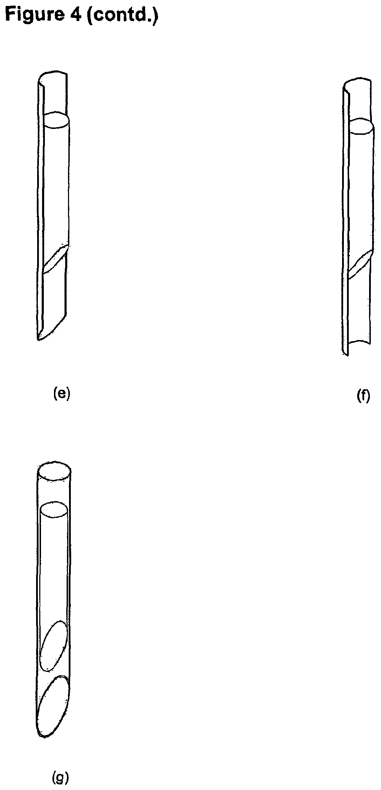

Figure 4 shows various different blades according to embodiments of the present invention, the blades are each shown with a solid drug formulation that is configured to match the shape of the blade. The figure shows blades which will provide different depth to length of cuts. A flat ended blade will give a constant depth cut across the whole length of the cut whereas a pointed blade will give a deeper cut in the middle of the cut length. The exact cut length to depth ratio may depend on the skin tissue target (area of body, gender, race, age etc) and / or the shape of tip of the solid dose formulation and / or the support required for the solid dose formulation in the skin.

### DETAILED DESCRIPTION OF THE DRAWINGS

### Figure 1

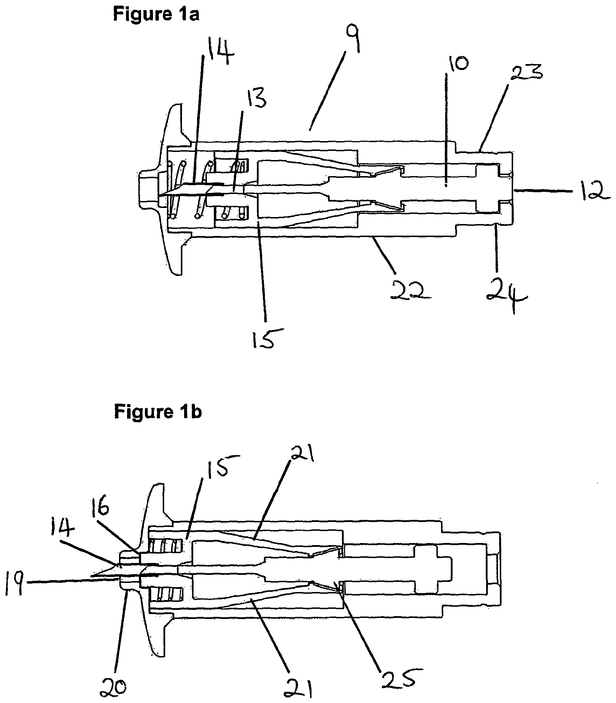

Figure 1a shows a cross sectional side view of the cassette portion of the solid dose injection device of the present invention. The cassette (9) is used with an actuation mechanism (not shown). Thus as can be seen in Figure 1a, in which the cassette includes a casing (22) having a narrower portion (23) that is inserted into the actuation mechanism prior to use, the grooves in the casing (24) helping to secure the two components. The actuation mechanism acts on the ejector pin (10) by striking the end of the ejector pin (12).

The cassette is shown prior to actuation and thus the blade (14), which in this particular embodiment is a hollow hypodermic needle, is held within the casing which reduces the risk of a sharps injury. The device comprises a single means for pushing the blade and the solid drug formulation, the single means being the ejector pin to which the blade mounting (15) is releasably coupled.

Figure 1b shows cassette upon actuation. To actuate the device the skin tensioning means (20) at the end of the casing is pushed against the skin. Upon actuation the actuation mechanism (not shown) strikes the end of the ejector pin (12) causing it to slide forwards inside the casing. The blade (14) is coupled, via resilient arms (21) of the blade mounting (15), to a wider portion (25) of the ejector pin (10), the ejector pin and the blade mounting both move forwards and the blade (14) is pushed forward out of aperture (19) and into the skin. The blade mounting (15) is stopped, in this embodiment by the end of the channel (16), and thus the blade, which is fixed to the blade mounting, can only travel a predetermined distance into the skin. Thus, the blade makes only a shallow cut in the skin.

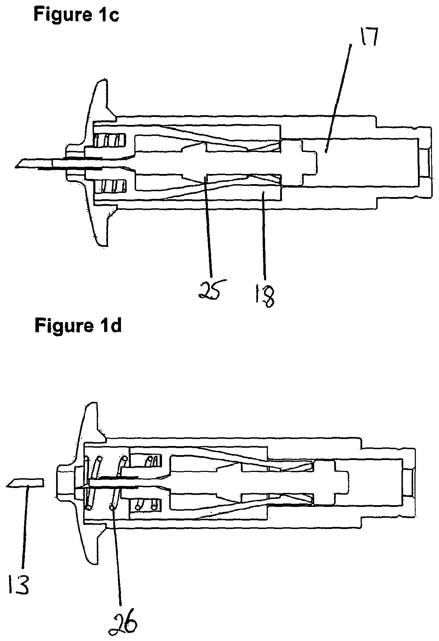

Figure 1c shows the next stage of the injection. The forward motion of the blade is stopped, the blade mounting (15) uncouples from the ejector pin and the ejector pin continues to move forwards, pushing the solid drug formulation into the cut in the skin. In this particular embodiment, the blade is in the form of the end of a hypodermic needle and thus the cassette is configured such that the ejector pin pushes the solid drug through the lumen of the needle. As can be seen in Figure 1c, the solid drug formulation (13) is pushed out beyond the depth of the blade. The ejector pin may be configured such that it travels along part of the length of the blade, along the full length or extends beyond the end of the blade.

The mechanism for uncoupling the blade mounting from the ejector pin is shown. As the ejector pin move forwards from the upper channel (17) into the wider lower channel (18) of the casing, the resilient arms (21) are able to flex and uncouple from the wider portion (25) of the ejector pin to which they are hooked.

As can be seen in the Figures, it is preferable that the ejector pin maintains contact with the solid drug formulation during injection so that the drug is pushed into the skin by the pin.

Figure 1d shows the final stage of the injection, the casing includes an automatic means for withdrawing the blade, which in this embodiment is in the form of a helical spring (26). As can be seen in Figure 1a, prior to actuation, the spring (26) is in a relaxed state and the blade is held within the casing. Upon actuation, the spring is compressed by the blade mounting and then the ejector pin. However, when the forward motion of the pin has stopped the spring is able to expand, forcing the ejector pin and blade mounting backwards and thus withdrawing the blade and the ejector pin from the skin.

### Figure 2

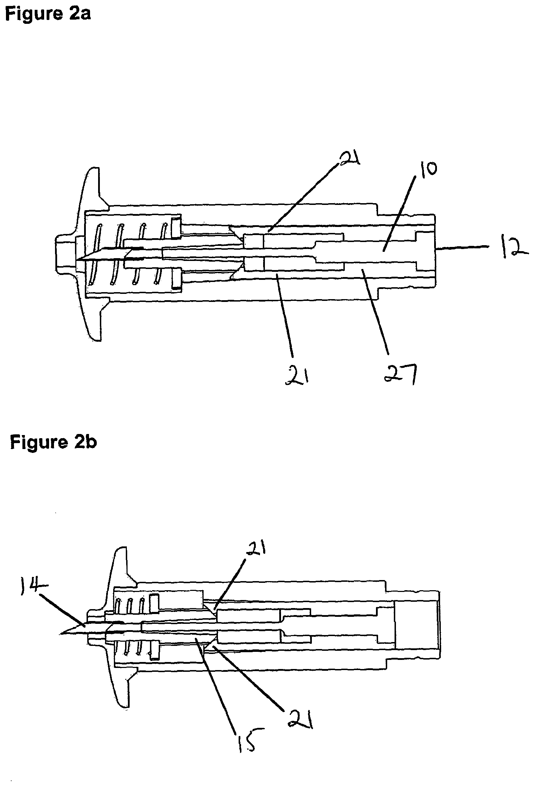

Figure 2a shows a cross sectional side view of the cassette portion of the solid dose injection device of the present invention showing an alternative means for coupling the blade mounting to the ejector pin. In this particular embodiment the movement of the ejector pin (10) is coupled to the movement of the blade mounting via resilient arms (21) on the ejector pin (10), which is mounted in an ejector pin mounting (27). The resilient arms may form an integral part of the ejector pin mounting.

### Figure 2b

Upon actuation the actuation mechanism capable of generating a force (not shown) strikes the end of the ejector pin (12). The ejector pin (10) comprises resilient arms which push against the end of the blade mounting (15) and thus both the blade mounting and ejector pin move forwards such that the blade (14) is inserted into the skin.

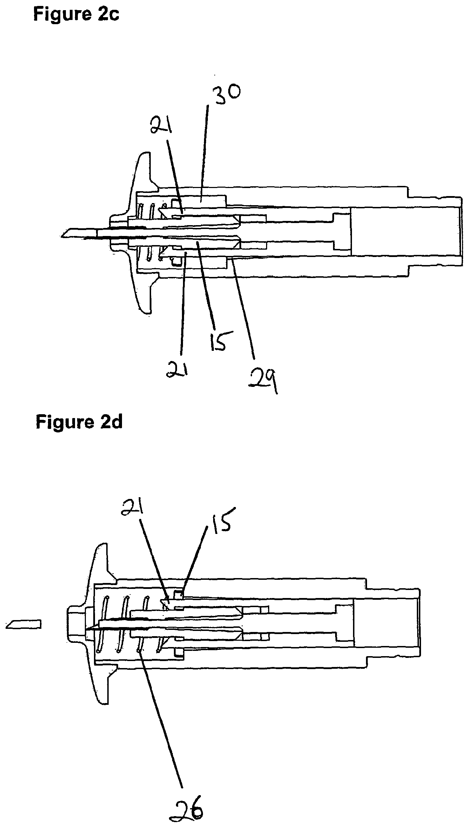

Figure 2c shows the next stage of the injection. The forward motion of the blade is stopped, in this embodiment by the end of the casing. The blade mounting (15) is uncoupled from the ejector pin (10) and the ejector pin continues to move forwards, pushing the solid drug formulation (13) into the cut in the skin.

The mechanism for uncoupling the blade mounting from the ejector pin is shown. As the ejector pin move forwards from the upper channel (29) into the wider lower channel (30) of the casing, the resilient arms (21) of the ejector pin mounting are able to flex and uncouple from the end of the blade mounting (15) and then ride over the end of the blade mounting.

### Figure 2d

Figure 2d shows the final stage of the injection, the casing includes an automatic means for withdrawing the blade, which in this embodiment is in the form of a helical spring (26). As can be seen in Figure 2a, prior to actuation, the spring (26) is in a relaxed state and the blade is held within the casing. Upon actuation of the device the spring is compressed by the blade mounting. However, when the forward motion of the pin has stopped, the spring is able to expand, the resilient arms of the ejector pin mounting hook over the blade mounting and thus both the ejector pin and blade mounting are forced backwards, withdrawing the blade and the ejector pin from the skin.

### Figure 3

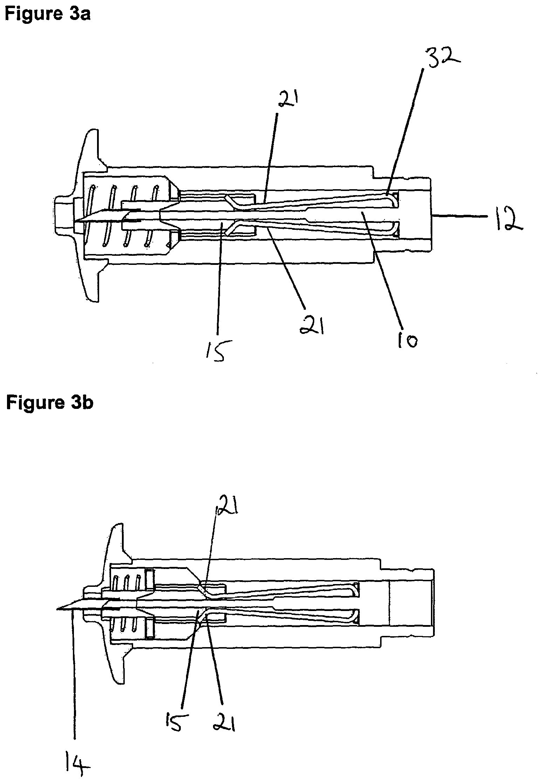

Figure 3a shows a cross sectional side view of the cassette portion of the solid dose injection device of the present invention showing an alternative means for coupling the blade mounting to the ejector pin. The device comprises a single means for pushing both the blade and the solid drug formulation, in the form of an ejector pin (10) to which the blade mounting (15) is releasably coupled. In this embodiment there are resilient arms mounted on the end of the ejector pin, the resilient arms are in the form of wire hairpin (32). The mechanism by which the wire arms couple and then uncouple from the blade mounting is similar to that shown in Figure 2.

### Figure 3b

Upon actuation, the actuation mechanism capable of generating a force (not shown) strikes the end of the ejector pin (12). The resilient arms (21) of the wire hairpin (32) push against the end of the blade mounting (15) and thus both the blade mounting and ejector pin move forwards such that the blade is inserted into the skin.

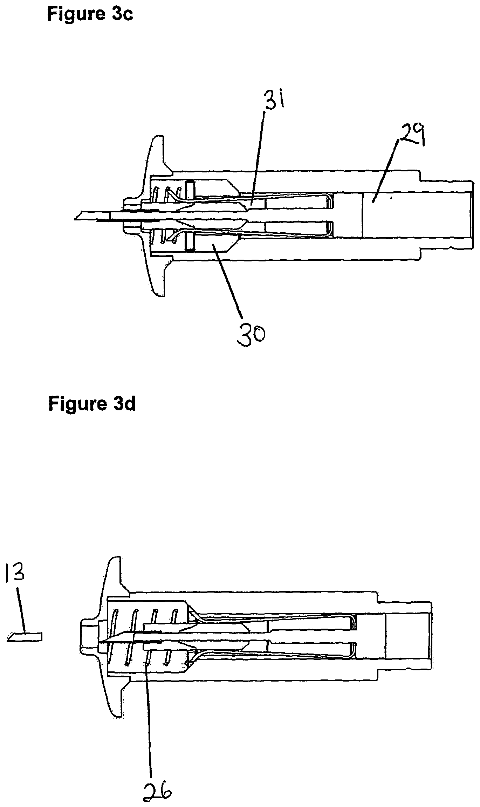

Figure 3c shows the next stage of the injection. The forward motion of the blade is stopped, in this embodiment by the end of the casing. The blade mounting (15) is uncoupled from the ejector pin and the ejector pin continues to move forwards, pushing the solid drug formulation into the cut in the skin.

The mechanism for uncoupling the blade mounting from the ejector pin is shown. As the ejector pin move forwards from the upper channel (29) into the wider lower channel (30) of the casing, the resilient arms of the wire hairpin are able to flex and uncouple from the end (31) of the blade mounting (15) and then ride over the end of the blade mounting.

Figure 3d shows the final stage of the injection, the casing includes an automatic means for withdrawing the blade, which in this embodiment is in the form of a helical spring (26). As can be seen in Figure 3a, prior to actuation, the spring (26) is in a relaxed state and the blade is held within the casing. Upon actuation of the device the spring is compressed by the blade mounting. However, when the forward motion of the pin has stopped, the spring is able to expand, the resilient arms (21) of the wire hairpin press against the blade mounting and thus both the ejector pin and blade mounting are forced backwards, withdrawing the blade and the ejector pin from the skin.

Figure 4a shows a substantially flat blade with a pointed cutting end and a solid drug formulation having a substantially square cross-section. Thus, the face of the solid formulation that contacts the blade is configured to match the blade. The skin penetrating face of the solid drug formulation is pointed to facilitate its passage through the skin, enabling it to cut a path through the tissue beyond the cut made by the blade.

Figure 4b shows a substantially flat blade with a straight cutting end and a solid drug formulation having a substantially square cross-section. The sharp cutting edge of the blade can be seen.

Figures 4c and 4d show a substantially flat blade with a pointed cutting end and a solid drug formulation having a curved shape, in this particular embodiment it is a cylinder that is semi-circular in cross-section. Again, the face of the solid formulation that contacts the blade is configured to match the blade. The cutting edge of the blade may be pointed (Figure 4c) or straight (Figure 4d).

Figures 4e and 4f show curved blade that is semi-circular in cross-section with a pointed (Figure 4e) or a flat end (Figure 4f) with a curved shape solid drug formulation, that in this embodiment is cylindrical with an oblique skin penetrating face.

Figure 4g shows a needle-shaped blade with cylindrical implant inside - there is no Figure showing a corresponding flat ended blade, as such a blade might act like an 'apple-corer' creating a plug of tissue, rather than creating a cut that creates a pathway for the solid drug formulation.

The reference numerals given above are non-limiting but have been included solely for the purpose of assisting the reader.

A solid dose injection using the devices or methods (no methods are claimed) of the present disclosure would occur in a matter of milliseconds after actuation of the device and would seem instantaneous as far as the user is concerned. Thus, the injection can be performed quickly and, ideally, without the patient seeing the blade. It is believed that because only a shallow cut is made in the skin, the pain caused will be minimal and comparable to the sensation experienced by users of needle-free injection devices.

## Claims

1. A solid dose injection device comprising:
i) a casing (22);
ii) an actuation mechanism capable of generating a force;
iii) a blade (14);
iv) a solid drug formulation (13); and
v) means (10) for pushing said blade (14) and said solid drug formulation (13);
wherein the solid dose injection device is suitable to be actuated so that the actuation mechanism capable of generating a force acts on the means for pushing to push the blade (14) from the casing (22) to penetrate the skin of the human or animal body to a predetermined depth wherein, following skin penetration by the blade (14), the means (10) for pushing continues to push the solid drug formulation (13) out of the casing (22) and into the human or animal body; wherein the solid drug formulation (13) is pushed into the body to a depth that extends beyond the depth of the blade (14); and wherein said solid drug formulation (13) comprises a cutting element to facilitate its passage through the skin.

2. A solid dose injection device according to claim 1 wherein the blade penetrates the skin to a predetermined depth of between 2 and 4mm.

3. A solid dose injection device according to any one of the preceding claims wherein the blade is substantially flat, curved or hollow.

4. A solid dose injection device according to any of the preceding claims wherein the blade is a hollow needle.

5. A solid dose injection device according to claim 4 wherein the solid drug is pushed through the lumen of the needle into the body.

6. A solid dose injection device according to any one of the preceding claims comprising a single means for pushing the blade and solid drug formulation into the body.

7. A solid dose injection device according to Claim 6 wherein the single means comprises an ejector pin to which the blade is coupled via a blade mounting.

8. A solid dose injection device according to Claim 7 wherein the blade mounting is releasably coupled to the ejector pin.

9. A solid dose injection device according to any one of the preceding claims further comprising ejector pin retraction means for withdrawing the ejector pin following injection of the solid drug formulation.

10. A solid dose injection device according to any one of the preceding claims further comprising an automatic ejector pin retraction means.

11. A solid dose injection device according to the any of the preceding claims wherein a single retraction means withdraws the blade and the ejector pin.

12. A solid dose injection device according to any of the preceding claims wherein the device is a single-use device.

13. A solid dose injection device according to any of the preceding claims wherein the device comprises a disabling mechanism to prevent it being reused.

14. A solid dose injection device according to any one of Claims 1 to 13, wherein the actuation mechanism is reusable and wherein the device comprises a first part comprising a reusable actuation mechanism and a second part comprising a disposable cassette (9) housing the blade, the solid drug formulation and the means for pushing the blade and the solid drug form.

## Patentansprüche

1. Feststoffdosis-Injektionsvorrichtung, umfassend:
i) ein Gehäuse (22);
ii) einen Betätigungsmechanismus, der in der Lage ist, eine Kraft zu erzeugen;
iii) eine Klinge (14);
iv) eine Feststoff-Arzneimittelformulierung (13); und
v) Einrichtungen (10) zum Schieben der Klinge (14) und der Feststoff-Arzneimittelformulierung (13);
wobei die Feststoffdosis-Injektionsvorrichtung geeignet ist, um betätigt zu werden, sodass der Betätigungsmechanismus, der in der Lage ist, eine Kraft zu erzeugen, auf die Einrichtungen zum Schieben einwirkt, um die Klinge (14) aus dem Gehäuse (22) zu schieben, um in die Haut des menschlichen oder tierischen Körpers bis zu einer vorbestimmten Tiefe einzudringen, wobei die Klinge (14) nach Eindringen in die Haut durch die Einrichtung (10) zum Schieben fortfährt, die Feststoff-Arzneimittelformulierung (13) aus dem Gehäuse (22) und in den menschlichen oder tierischen Körper zu schieben; wobei die feste Feststoff-Arzneimittelformulierung(13) bis zu einer Tiefe in den Körper geschoben wird, die sich über die Tiefe der Klinge (14) hinaus erstreckt; und wobei die Feststoff-Arzneimittelformulierung (13) ein Schneideelement umfasst, um ihren Durchgang durch die Haut zu erleichtern.

2. Feststoffdosis-Injektionsvorrichtung nach Anspruch 1, wobei die Klinge die Haut bis zu einer vorbestimmten Tiefe von zwischen 2 und 4 mm eindringt.

3. Feststoffdosis-Injektionsvorrichtung nach einem der vorherigen Ansprüche, wobei die Klinge im Wesentlichen flach, gekrümmt oder hohl ist.

4. Feststoffdosis-Injektionsvorrichtung nach einem der vorherigen Ansprüche, wobei die Klinge eine Hohlnadel ist.

5. Feststoffdosis-Injektionsvorrichtung nach Anspruch 4, wobei das Feststoffarzneimittel durch das Lumen der Nadel in den Körper geschoben wird.

6. Feststoffdosis-Injektionsvorrichtung nach einem der vorherigen Ansprüche, umfassend eine einzige Einrichtung zum Einschieben der Klinge und der Feststoff-Arzneimittelformulierung in den Körper.

7. Feststoffdosis-Injektionsvorrichtung nach Anspruch 6, wobei die einzelne Einrichtung einen Auswerferstift umfasst, an den die Klinge über eine Klingenhalterung gekoppelt ist.

8. Feststoffdosis-Injektionsvorrichtung nach Anspruch 7, wobei die Klingenhalterung lösbar mit dem Auswerferstift gekoppelt ist.

9. Feststoffdosis-Injektionsvorrichtung nach einem der vorherigen Ansprüche, ferner umfassend eine Auswerferstift-Rückzugseinrichtung zum Zurückziehen des Auswerferstifts nach Injektion der Feststoff-Arzneimittelformulierung.

10. Feststoffdosis-Injektionsvorrichtung nach einem der vorherigen Ansprüche, ferner umfassend eine automatische Auswerferstift-Rückzugseinrichtung.

11. Feststoffdosis-Injektionsvorrichtung nach einem der vorherigen Ansprüche, wobei eine einzelne Rückzugseinrichtung die Klinge und den Auswerferstift zurückzieht.

12. Feststoffdosis-Injektionsvorrichtung nach einem der vorherigen Ansprüche, wobei die Vorrichtung eine Einwegvorrichtung ist.

13. Feststoffdosis-Injektionsvorrichtung nach einem der vorherigen Ansprüche, wobei die Vorrichtung einen Deaktivierungsmechanismus umfasst, um ihre Wiederverwendung zu verhindern.

14. Feststoffdosis-Injektionsvorrichtung nach einem der Ansprüche 1 bis 13, wobei der Betätigungsmechanismus wiederverwendbar ist und wobei die Vorrichtung einen ersten Teil, umfassend einen wiederverwendbaren Betätigungsmechanismus, und einen zweiten Teil, umfassend eine Einwegkassette (9), die die Klinge, die Feststoff-Arzneimittelformulierung und die Einrichtungen zum Schieben der Klinge und das Feststoff-Arzneimittelformular enthält, umfasst.

## Revendications

1. Dispositif d'injection de dose solide comprenant :
i) un boîtier (22) ;
ii) un mécanisme d'actionnement pouvant générer une force ;
iii) une lame (14) ;
iv) une formulation de médicament solide (13) ; et
v) un moyen (10) destiné à pousser ladite lame (14) et ladite formulation de médicament solide (13) ;
ledit dispositif d'injection de dose solide étant conçu pour être actionné de sorte que le mécanisme d'actionnement pouvant générer une force agisse sur le moyen de poussée pour pousser la lame (14) hors du boîtier (22) afin de pénétrer dans la peau du corps humain ou animal à une profondeur prédéfinie ; suite à la pénétration cutanée de la lame (14), ledit moyen (10) de poussée continuant de pousser la formulation de médicament solide (13) hors du boîtier (22) et dans le corps humain ou animal ; ladite formulation de médicament solide (13) étant poussée dans le corps jusqu'à une profondeur qui s'étend au-delà de la profondeur de la lame (14) ; et ladite formulation de médicament solide (13) comprenant un élément coupant destiné à faciliter son passage à travers la peau.

2. Dispositif d'injection de dose solide selon la revendication 1, ladite lame pénétrant dans la peau jusqu'à une profondeur prédéfinie comprise entre 2 et 4 mm.

3. Dispositif d'injection de dose solide selon l'une quelconque des revendications précédentes, ladite lame étant sensiblement plate, incurvée ou creuse.

4. Dispositif d'injection de dose solide selon l'une quelconque des revendications précédentes, ladite lame étant une aiguille creuse.

5. Dispositif d'injection de dose solide selon la revendication 4, ledit médicament solide étant poussé à travers la lumière de l'aiguille dans le corps.

6. Dispositif d'injection de dose solide selon l'une quelconque des revendications précédentes comprenant un seul moyen destiné à pousser la lame et la formulation de médicament solide dans le corps.

7. Dispositif d'injection de dose solide selon la revendication 6, ledit seul moyen comprenant une broche d'éjection à laquelle la lame est couplée par l'intermédiaire d'un support de lame.

8. Dispositif d'injection de dose solide selon la revendication 7, ledit support de lame étant couplé de manière amovible à la broche d'éjection.

9. Dispositif d'injection de dose solide selon l'une quelconque des revendications précédentes, comprenant en outre un moyen de rétraction de broche d'éjection destiné à retirer la broche d'éjection après injection de la formulation de médicament solide.

10. Dispositif d'injection de dose solide selon l'une quelconque des revendications précédentes, comprenant en outre un moyen de rétraction automatique de broche d'éjection.

11. Dispositif d'injection de dose solide selon l'une quelconque des revendications précédentes, un seul moyen de rétraction retirant la lame et la broche d'éjection.

12. Dispositif d'injection de dose solide selon l'une quelconque des revendications précédentes, ledit dispositif étant un dispositif à usage unique.

13. Dispositif d'injection de dose solide selon l'une quelconque des revendications précédentes, ledit dispositif comprenant un mécanisme de neutralisation pour empêcher sa réutilisation.

14. Dispositif d'injection de dose solide selon l'une quelconque des revendications 1 à 13, ledit mécanisme d'actionnement étant réutilisable et ledit dispositif comprenant une première partie comprenant un mécanisme d'actionnement réutilisable et une seconde partie comprenant une cassette jetable (9) hébergeant la lame, la formulation de médicament solide et le moyen destiné à pousser la lame et la forme médicamenteuse solide.
